# EUROPEAN PATENT APPLICATION

(11) **EP 4 497 407 A1**
(43) Date of publication of application: **29.01.2025**
(21) Application number: 23795072.0
(22) Date of filing: 14.04.2023
(51) Int. Cl.: A61B 34/30, A61B 17/32

(54) **CONTROL MECHANISM OF SURGICAL INSTRUMENT AND SURGICAL ROBOT**

(30) Priority: 29.04.2022 CN 202210474834
(71) Applicant: Cornerstone Technology (Shenzhen) Limited, Shenzhen Guandong 518066 (CN)
(72) Inventor: YANG, Qiusheng, Shenzhen, Guangdong 518000 (CN); ZHANG, Jianwei, Shenzhen, Guangdong 518000 (CN); WANG, Zerui, Shenzhen, Guangdong 518000 (CN)
(74) Representative: Balder IP Law, S.L.
(86) International application number: PCT/CN2023/088505
(87) International publication number: WO 2023/207639

(57) **Abstract**

The present disclosure discloses a control mechanism of a surgical instrument and a surgical robot. The control mechanism of the surgical instrument is connected to an effector of the surgical instrument and is configured to control a clamp assembly of the effector to open or close. The control mechanism of the surgical instrument includes a base, an operation assembly connected to the effector, a first transmission assembly arranged on the base, and a lever having a first end, a second end and a lever body. The first transmission assembly is rotatable under control of a drive mechanism. The first end of the lever is connected to the first transmission assembly and is movable vertically with rotation of the first transmission assembly. The second end is connected to the operation assembly. The lever body is pivotably connected to the base to form a fulcrum. An upward movement of the first end causes the clamp assembly to open; and a downward movement of the first end causes the clamp assembly to close.

## Description

### CROSS REFERENCE TO RELATED APPLICATIONS

The present application claims priority to Chinese patent application No. 202210474834.0, entitled "CONTROL MECHANISM OF SURGICAL INSTRUMENT AND SURGICAL ROBOT," filed on April 29, 2022, which is incorporated by reference herein in its entirety.

### TECHNICAL FIELD

The present disclosure relates to the field of medical instrument technology, and in particular to a control mechanism of a surgical instrument and a surgical robot.

### BACKGROUND

In minimally invasive surgery, doctors often need to manually cut, peel, and suture tissues. In order to reduce the workload of doctors and minimize bleeding or wounds of patients, ultrasound tools are increasingly being used in surgery. The principle of the ultrasound tools is commonly to convert ultrasound energy or transmit ultrasound energy into biological tissues using surgical instruments, thereby generating physiological effects, and utilizing the generated heat to burn the tissues to stop bleeding or to cut the tissues. For example, in some ultrasound tools of surgical instruments, a generator generates high-frequency electrical energy, a transducer converts the high-frequency energy into mechanical vibration using piezoelectric materials or electromagnetic compression materials, and the vibration is amplified and transmitted to an end effector, in order to achieve burning or cutting.

At least some of the existing surgical robots and ultrasound tools have complex engagement structures, which do not facilitate the manual operation in special situations.

### SUMMARY

In the summary, a series of simplified concepts are introduced, which will be specified in the detailed description of the embodiments. The summary of the present disclosure does not imply trying to limit key technical features and essential technical features of the claimed technical solutions, nor imply trying to limit the scope of protection of the claimed technical solutions.

A first aspect of embodiments of the present disclosure provides a control mechanism of a surgical instrument. The control mechanism of the surgical instrument includes a base, an operation assembly configured to be connected to an effector of the surgical instrument, a first transmission assembly arranged on the base and configured to engage with a drive mechanism, and a lever having a first end, a second end opposite to the first end and a lever body. The first transmission assembly is rotatable under control of the drive mechanism. The first end of the lever is connected to the first transmission assembly and is movable vertically with rotation of the first transmission assembly. The second end of the lever is connected to the operation assembly. The lever body of the lever is pivotably connected to the base to form a fulcrum. The control mechanism is configured to control a clamp assembly of the effector of the surgical instrument, such that: an upward movement of the first end of the lever causes the clamp assembly to open; and a downward movement of the first end of the lever causes the clamp assembly to close.

In some embodiments, the first transmission assembly includes a first transmission disc and a rotating rod having threads. The first transmission disc is rotatably connected to a bottom of the base, the rotating rod is fixed and connected on the first transmission disc, and the first end of the lever acts on the rotating rod. The base has a front end and a rear end opposite to each other, a second instrument assembly is arranged at the front end, the first transmission disc is arranged at the rear end, and a notch is defined on the rear end of the base to expose the first transmission disc.

In some embodiments, the control mechanism of the surgical instrument further includes a support seat arranged on the base, and the support seat is pivotably connected to an upper portion of the rotating rod.

In some embodiments, the control mechanism of the surgical instrument further includes a manual control part arranged at a top end of the rotating rod, and the support seat defines an opening corresponding to the manual control part. The control mechanism of the surgical instrument further includes a housing covering on the base, and the housing defines a through hole corresponding to the manual control part and the opening.

In some embodiments, the first end of the lever has a first connection portion configured as a recess for receiving the rotating rod, the threads of the rotating rod are configured as a cooperation part engaged with a connection part arranged on the first connection portion, and rotation of the rotating rod render an upward movement or a downward movement of the first connection portion, along spiral cooperation part.

In some embodiments, the rotating rod is configured as a threaded rod. The control mechanism of the surgical instrument further includes a lifting assembly threadedly engaged with the threaded rod, and rotation of the threaded rod renders an upward movement or a downward movement of the lifting assembly. The first end of the lever is connected to the threaded rod via the lifting assembly.

In some embodiments, the first end of the lever has a first connection portion, the lifting assembly includes a cooperation part corresponding to the first connection portion, the cooperation part is movably connected to the first connection portion to provide the lever with a horizontal movement margin.

In some embodiments, one of the cooperation part and the first connection portion is configured as a protrusion, and an other of the cooperation part and the first connection portion is configured as a recess fitted with the protrusion. At least one kidney-shaped slot is defined on a side of one of the cooperation part and the first connection portion, a connection part is arranged on a side of an other of the cooperation part and the first connection portion, and the connection part extends into the at least one kidney-shaped slot to form a hinge connection. A length direction of the at least one kidney-shaped slot intersects with a vertical direction to form the horizontal movement margin.

In some embodiments, the connection part is configured as a cylindrical rod or a bearing slidable or rollable in the at least one kidney-shaped slot.

In some embodiments, the control mechanism of the surgical instrument further includes a link arranged between the cooperation part and the first connection portion, and each of the cooperation part and the first connection portion is pivotally connected with the link.

In some embodiments, the cooperation part has first teeth arranged vertically, the first connection portion has second teeth, the second teeth are arranged on a surface having a circular arc shape centered at the fulcrum of the lever, and the first teeth mesh with the second teeth.

In some embodiments, a guide groove is defined on the support seat and extends vertically, and the cooperation part extends out of the support seat via the guide groove.

In some embodiments, the control mechanism of the surgical instrument further includes a second transmission assembly configured to engage with the drive mechanism. The second transmission assembly is rotatable under control of the drive mechanism, and the second transmission assembly is configured to be connected to the effector to drive the effector to rotate with the second transmission assembly.

In some embodiments, the second transmission assembly includes a second transmission disc and a first gear, the second transmission disc is arranged on a bottom of the base, and the first gear is fixed and connected on the second transmission disc. The first gear is configured to mesh with a second gear connected to the effector.

In some embodiments, the operation assembly includes an operation portion, and a periphery of the operation portion defines a circumferential engagement groove. The second end of the lever has a second connection portion at least partially surrounding the operation portion. The second connection portion has an extension portion at least partially extending into the engagement groove. The extension portion has a size fitting with a size of the engagement groove to enable a vertical movement of the operation portion with pivoting of the second connection portion and enable rotation of the operation portion.

In some embodiments, the second connection portion includes a first arm and a second arm spaced from each other, the operation portion is arranged between the first arm and the second arm, the first arm has a first protrusion, the second arm has a second protrusion, and each of the first protrusion and the second protrusion at least partially extends into the engagement groove; or the second connection portion is configured as an annular portion surrounding the operation portion, the annular portion has the extension portion arranged circumferentially, and the extension portion extends into the engagement groove.

In some embodiments, the operation assembly further includes a traction portion configured to be connected to the effector, a limiting portion and an elastic part. The operation portion is sleeved on the traction portion, and the operation portion is vertically moveable relative to the traction portion. The limiting portion is arranged on a top of the traction portion and on an upper side of the operation portion. The elastic part is sleeved on the traction portion and arranged between the limiting portion and the operation portion.

In some embodiments, the lifting assembly is provided with a sensed element, the base is provided with a sensing device configured to sense positions of the sensed element, and the positions of the sensed element corresponds to states of the clamp assembly.

In some embodiments, the sensed element is configured as a pressing block, the sensing device has a pressure switch, and the pressing block is configured to press on the pressure switch when the clamp assembly is closed.

In some embodiments, the surgical instrument is configured as an ultrasound tool, and a blade and a jaw of the ultrasound tool form the clamp assembly.

The control mechanism of the surgical instrument according to embodiments of the present disclosure uses a lever structure for transmission between rotation movement and vertical movement. This lever structure is relatively simple, and has low failure rate and low cost. This lever structure results in less redundancy in transmission, which is conducive to precise control of clamping of an ultrasound tool. Moreover, the first transmission disc is arranged to be far away from the ultrasound tool, which is conducive to manual operation of the first transmission disc, thereby facilitating the installation and cleaning of the ultrasound tool.

A second aspect of embodiments of the present disclosure provides a surgical robot including the control mechanism of the surgical instrument according to the first aspect as illustrate above.

The surgical robot according to the embodiments of the present disclosure can achieve technical effects similar to those of the control mechanism of the surgical instrument according to the first aspect as illustrate above.

### BRIEF DESCRIPTION OF THE DRAWINGS

The following drawings of the present disclosure are used, as a part of the present disclosure, for understanding of the present disclosure. The accompanying drawings illustrate embodiments and their descriptions of the present disclosure to illustrate the principles of the present disclosure.
FIG. 1 is a schematic diagram of a control mechanism of a surgical instrument according to some embodiments of the present disclosure.
FIG. 2 is a perspective view of a structure of a control mechanism of a surgical instrument according to a first embodiment of the present disclosure.
FIG. 3 is a perspective view of the structure of the control mechanism of the surgical instrument from another perspective in FIG. 2.
FIG. 4 is a top view of the control mechanism of the surgical instrument in FIG. 2.
FIG. 5 is a side view of the control mechanism of the surgical instrument in FIG. 2.
FIG. 6 is a sectional view of the control mechanism of the surgical instrument in FIG. 2.
FIG. 7 is another perspective view of a structure of a control mechanism of a surgical instrument according to a second embodiment of the present disclosure.
FIG. 8 is a side view of the control mechanism of the surgical instrument in FIG. 7.
FIG. 9 is a sectional view of the control mechanism of the surgical instrument in FIG. 7.
FIG. 10 is still another perspective view of a structure of a control mechanism of a surgical instrument according to a third embodiment of the present disclosure.

### DETAILED DESCRIPTION OF THE EMBODIMENTS

In the following description, a large number of details are provided for more thorough understanding of the present disclosure. However, it is obvious to those skilled in the art that the present disclosure can be implemented without one or more of these details. In other examples, in order to prevent confusion with the present disclosure, some well-known technical features in the art are not described.

In order to fully understand the present disclosure, a detailed description will be provided in the following. It should be understood that providing these embodiments is to make the present disclosure thorough and complete, and to fully convey the ideas of these exemplary embodiments to those skilled in the art. Obviously, the implementation of the embodiments of the present disclosure is not limited to specific details familiar to those skilled in the art. The preferred embodiments of the present disclosure are described in detail hereinafter, however, in addition to these detailed descriptions, the present disclosure may further include other embodiments.

It should be noted that the terms used herein are only for describing the embodiments and are not intended to limit exemplary embodiments of the present disclosure. As used herein, unless otherwise explicitly stated in the context, the singular form is also intended to include the plural form. In addition, it should be understood that when the terms "comprising" and/or "including" are used in the description, they indicate the existence of the features, entirety, steps, operations, elements and/or components, but do not exclude the existence or addition of one or more other features, entirety, steps, operations, elements and/or components and/or their combination.

The ordinal numerals such as "first" and "second" referred to in the present disclosure are merely identifiers and do not have any other meaning, such as implying a specific order. Moreover, for example, the term "first component" does not imply the existence of a "second component", and the term "second component" does not imply the existence of a "first component". It should be noted that the terms "up", "down", "front", "back", "left", "right", "inside", "outside" and the like used in the present disclosure are for illustrative purposes only and are not restrictive.

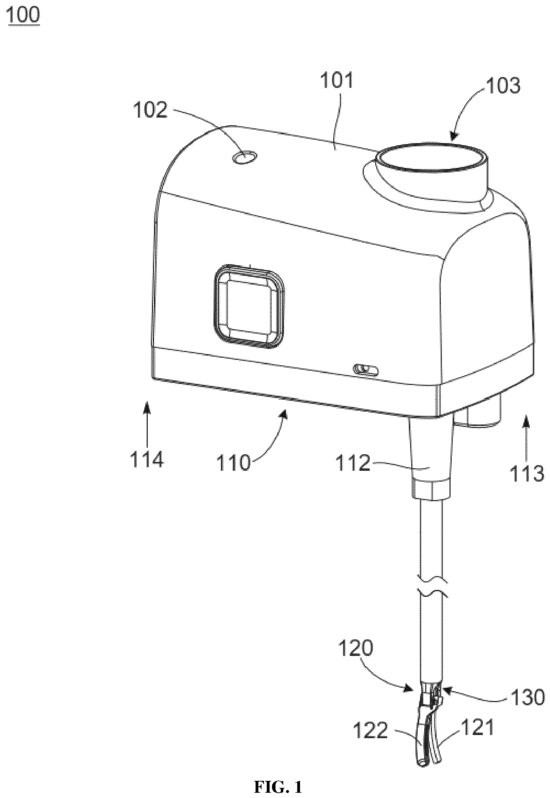

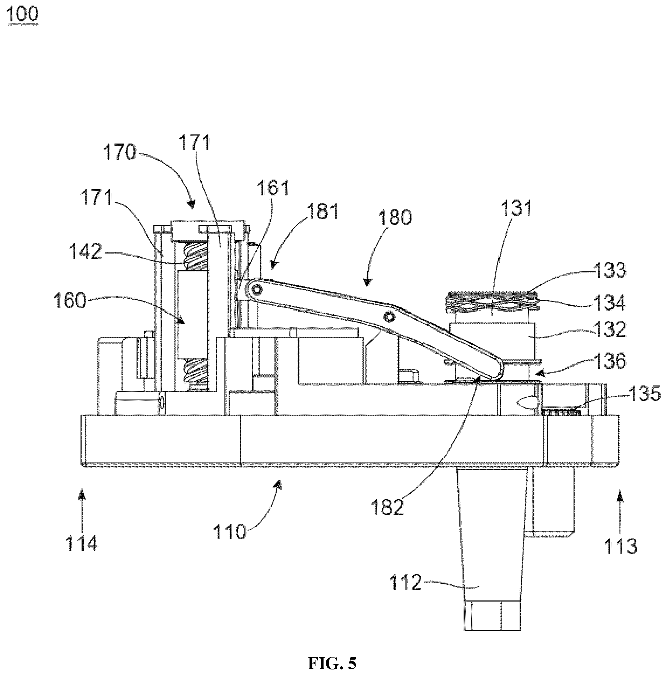

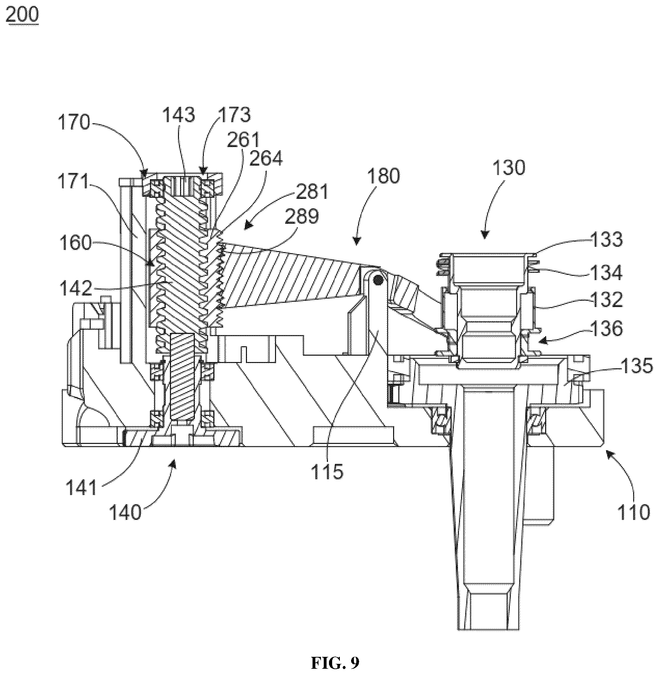

Exemplary embodiments of the present disclosure will be illustrated in detail referring to FIGS. 1 to 10.

Referring to FIGS. 1 to 6, a first embodiment of the present disclosure provides a control mechanism 100 of a surgical instrument. The surgical instrument has an effector including a first instrument assembly 120, a second instrument assembly 130 and a clamp assembly. One of the first instrument assembly 120 and the second instrument assembly 130 is fixed, and the other one is movable vertically. When one of the first instrument assembly 120 and the second instrument assembly 130 moves upwards, the clamp assembly tends to be closed, and when the one of the first instrument assembly 120 and the second instrument assembly 130 moves downwards, the clamp assembly tends to be opened. Moreover, the second instrument assembly 130 is not movable in a circumferential direction relative to the first instrument assembly 120. That is to say, the second instrument assembly 130 and the first instrument assembly 120 are further configured to rotate synchronously.

In some embodiments, the surgical instrument may be an ultrasound tool. The first instrument assembly 120 includes a transmission rod and a fixing sleeve. The transmission rod is constructed as an elongated solid rod, and a blade 121 is arranged on a bottom end of the transmission rod. The fixing sleeve and the transmission rod are fixed by a bolt. The transmission rod is configured to connect to a transducer to transmit energy to the blade 121. The second instrument assembly 130 may be a traction sleeve sleeved on the transmission rod and disposed between the transmission rod and the fixing sleeve, or sleeved on the fixing sleeve. In addition, a kidney-shaped slot extending vertically is defined on the traction sleeve, and the bolt is inserted into the kidney-shaped slot to prevent the transmission rod from rotation relative to the traction sleeve, in such a way that synchronous rotation of the transmission rod, the fixing sleeve, and the traction sleeve about an axial direction is achieved. The bolt and the kidney-shaped slot are used to provide space for the vertical movement of the traction sleeve relative to the transmission rod. In some embodiments, a jaw 122 is pivotally connected to a bottom end of the second instrument assembly 130, and the jaw 122 has a non-clamping portion with two different positions pivotally connected with the fixing sleeve and the traction sleeve, respectively, so that the vertical movement of the traction sleeve can drive the jaw 122 to open or close relative to the blade, in order to implement the opening and closing of the jaw 122 and the blade 121 with the vertical movement of the second instrument component 130. It can be understood that the jaw 122 and the blade 121 form the aforementioned clamp assembly.

The control mechanism 100 of the surgical instrument includes a base 110, a housing 101, an operation assembly, a first transmission assembly 140, a lifting assembly 160, a lever 180 and a second transmission assembly 150.

The housing 101 is arranged on the base 110. The base 110 has a front end 113 and a rear end 114. A sleeve 112 is arranged on the base 110, close to the front end 113. The above surgical instrument (ultrasound tool) is arranged in the sleeve 112 to penetrate the base 110. The housing 101 is provided with a transducer port 103 (as shown in FIG. 1), the wire of the transducer can run through the transducer port 103 and connect to the first instrument assembly 120 (e.g., the transmission rod) of the surgical instrument.

The operation assembly is arranged on a top of the first instrument assembly 120 and/or a top of the second instrument assembly 130. In some embodiments, the operation assembly is arranged on the top of the second instrument assembly 130.

The second transmission assembly 150 is arranged to be close to the sleeve 112 and includes a second transmission disc 151 and a first gear 152. The second transmission disc 151 is arranged on a bottom of the base 110, the first gear 152 is fixed and connected on the second transmission disc 151, and the first gear and the second transmission disc are coaxial. The second transmission disc 151 is configured to be engage with a drive mechanism and can rotate under control of the drive mechanism, and further drives the first gear 152 to rotate synchronously. A second gear 135 is fixed and connected on a periphery of the second instrument assembly 130, and the second gear meshes with the first gear 152. Thus, the second instrument assembly 130 can rotate with rotation of the second transmission assembly 150, thereby driving the first instrument assembly 120 to rotate together.

The first transmission assembly 140 is arranged to be close to the rear end 114 of the base 110 and includes a first transmission disc 141 and a rotating rod 142. The first transmission disc 141 is rotatably arranged on the bottom of the base 110 and is configured to engage with the drive mechanism. The rotating rod 142 is fixed and connected on the first transmission disc 141, and the first transmission disc and the rotating rod are coaxial. The first transmission disc and the rotating rod may be constructed as one piece or several separate pieces that are connected. Thus, the first transmission disc 141 can drive the rotating rod 142 to rotate under control of the drive mechanism.

In some embodiments, the rotating rod 142 is configured as a threaded rod having male threads. The lifting assembly 160 is accordingly configured to have female threads and is sleeved on the threaded rod with a threaded connection between the lifting assembly 160 and the rotating rod 142. The control mechanism of the surgical instrument further includes a support seat 170 arranged on the base 110 and arranged to correspond to the first transmission assembly 140. For example, the support seat 170 is arranged on an upper portion of the rotating rod 142, and the support seat 170 is pivotably connected to the upper portion of the rotating rod 142. In some embodiments, the support seat 170 is connected to the rotating rod 142 via a bearing. A guide groove 172 is defined on a side of the support seat 170 and extends vertically. The lifting assembly 160 is provided, on a side of it, with a cooperation part 161 extending out of the support seat via the guide groove 172, so that when the rotating rod 142 rotates, the lifting assembly 160 can move vertically instead of rotating with the rotation of the rotating rod. For example, the support seat 170 includes several legs 171 connected to the base 110, such as three legs 171 in the embodiment, and a space between two legs 171 forms the above-mentioned guide groove 172.

The lever 180 has a first end, a second end opposite to the first end and a lever body. The first end is connected to the cooperation part 161 of the lifting assembly 160 and is movable vertically with the lifting assembly 160. The second end is connected to the operation assembly, and the lever body of the lever 180 is pivotably connected to the base 110. In this way, the pivot between the lever 180 and the base 110 forms a fulcrum of a lever structure, such that when the first end of the lever 180 move upwards, the second instrument assembly 130 moves downwards, thereby causing the clamp assembly to open, namely the jaw 122 pivots in an opening direction. When the first end of the lever 180 move downwards, the second instrument assembly 130 moves upwards, thereby causing the clamp assembly to close, namely the jaw 122 pivots in a closing direction.

In some embodiments, a through groove 187 is defined in the middle of the lever 180, and a fulcrum part 115 is arranged on the base 110. The fulcrum part 115 extends into the through groove 187 and is pivotably connected on a lateral surface of the through groove 187. In an embodiment not shown, the groove through 187 may not be employed in the lever 180, but a top of the fulcrum part 115 has a forked shape to form a recess, in order to accommodate the lever 180, and the forked top of the fulcrum part is pivotably connected to the side surfaces of the lever 180.

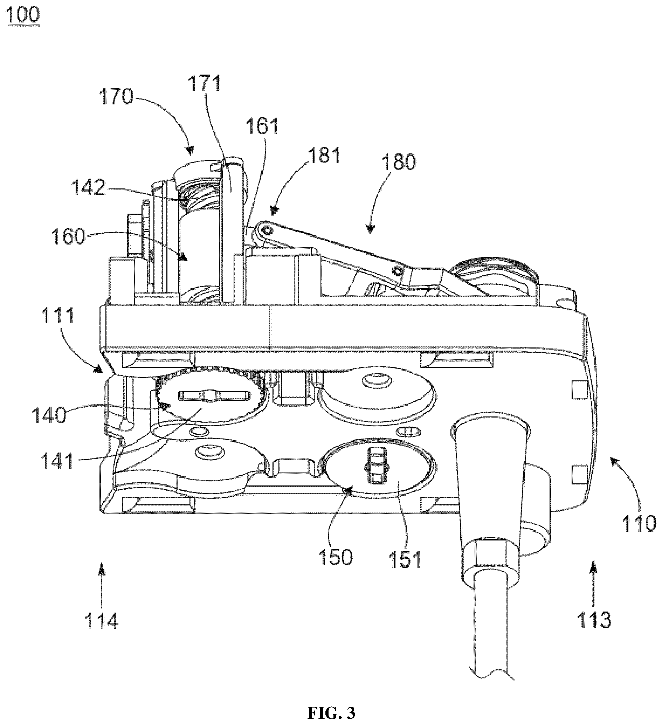

Referring to FIG. 3, due to the fact that when installing the ultrasound tool to the surgical robot or removing the ultrasonic tool from the surgical robot, the jaw 122 and the blade 121 need to be manually closed, a notch 111 may be defined on the rear end 114 of the base 110 to expose the first transmission disc 141, in order to facilitate manual operation. In this way, users can directly manually operate the first transmission disc 141 to control the opening and closing of the jaw 122.

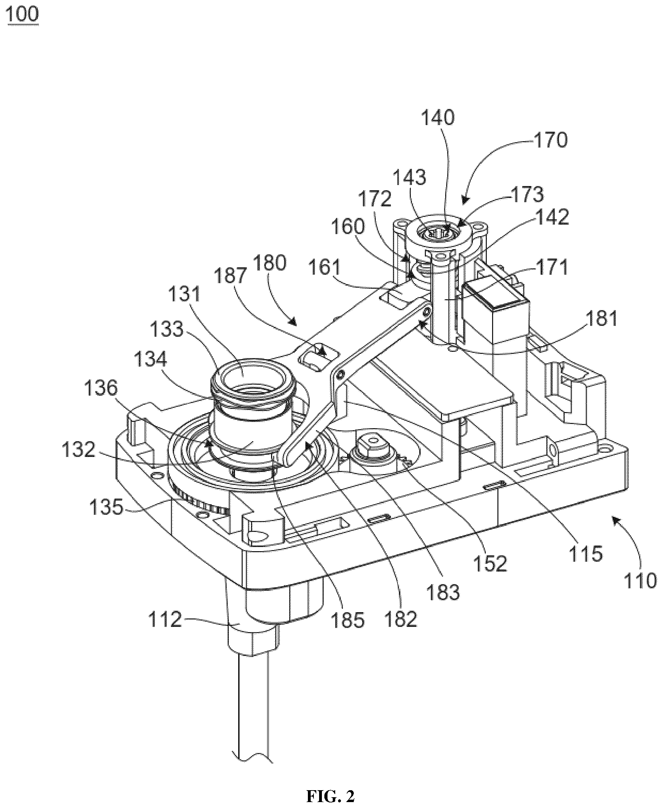

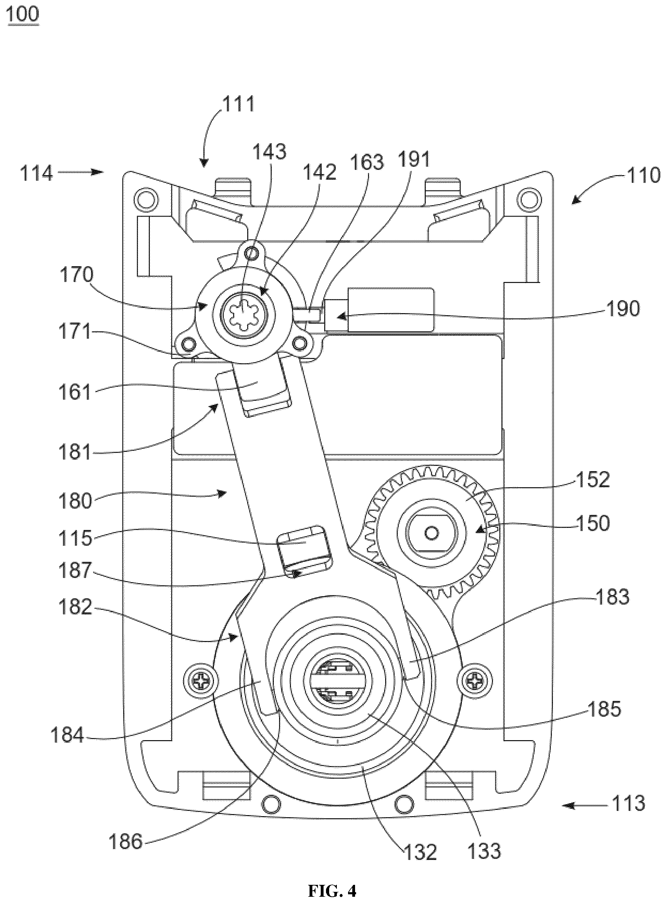

In order to further facilitate manual operation of users, in some embodiments, the control mechanism of the surgical instrument further includes a manual control part 143 (as shown in FIGS. 2 and 4) arranged at a top end of the rotating rod 142. For example, the manual control part 143 may be configured as a pattern, a hole, a straight screw slot, a cross screw slot, or the like that are matched with corresponding tools. Accordingly, the support seat 170 defines an opening 173, and the housing 101 defines a through hole 102 (as shown in FIG. 1) over the opening 173. In this way, users can use tools to operate the manual control part 143 through the through-hole 102 on the housing and the opening 173 on the support seat 170, thereby directly controlling the rotation of the rotating rod 142 to control the opening and closing of the clamp assembly.

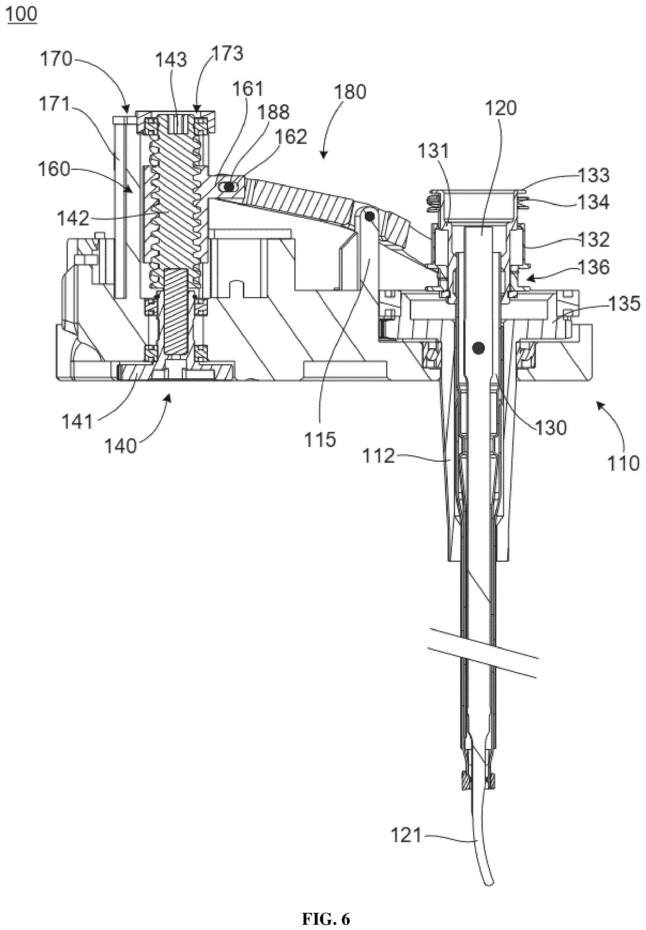

The following will provide a detailed description of the connection structures of the lever 180 and the lifting assembly 160, in conjunction with FIG. 6. The first end of the lever 180 has a first connection portion 181 configured as a recess. Accordingly, the cooperation part 161 is configured as a protrusion convex relative to the lifting assembly 160, and the convex cooperation part 161 is accommodated in the concave first connection portion 181.

At least one kidney-shaped slot 162 is defined on a side of the cooperation part 161. The at least one kidney-shaped slot 162 may be a through hole 102 penetrating through the cooperation part 161, or may be blind holes defined on both sides of the cooperation part 161, respectively. Moreover, a length direction of the at least one kidney-shaped slot 162 intersects with a vertical direction to form the horizontal movement margin. In some embodiments, the length direction of the at least one kidney-shaped slot 162 is in a horizontal plane. The two branches of the recess of the first connection portion 181 are provided with connection parts 188 facing inwards, respectively, and the connection parts 188 arranged on the two branches extend inwards and into corresponding kidney-shaped slot(s) 162, respectively, in order to form a hinge connection, such that the connection parts 188 are moveable in the at least one kidney-shaped slot 162. In this way, the first connection portion 181 can smoothly move vertically with the vertical movement of the lifting assembly 160 without being stuck. In some embodiments, the connection parts 188 may be configured as cylindrical rods or bearings.

The following will provide a detailed description of the connection structures of the lever 180 and the operation assembly, in conjunction with FIGS. 2, 4, 5 and 6. The operation assembly includes a traction portion 131, an operation portion 132, a limiting portion 133, and an elastic part 134.

The traction portion 131 is connected to a top of the second instrument assembly 130. In some embodiments, the traction portion 131 is sleeved on the second instrument assembly 130, in order to reserve a channel for the wiring of the transducer which is connected into the second instrument assembly 130.

The operation portion 132 is sleeved on the traction portion 131, and the operation portion 132 is configured to be moveable, for example slidable, vertically relative to the traction portion 131. The limiting portion 133 is arranged on a top of the traction portion and is configured to protrude outwards, and the elastic part 134 is arranged between the operation portion 132 and the limiting portion 133 and may be connected to both the operation portion and the limiting portion, in order to apply elastic force to the operation portion 132 that makes the operation portion to tend to move downwards. In some embodiments, the elastic part 134 may be a compression spring. In this way, when the jaw 122 and the blade 121 are closed, but the second end of the lever 180 still tends to move upwards, a buffer can be formed by the structure composed of the operation portion 132, the limiting portion 133 and the elastic part 134 to protect the blade 121 from damage due to excessive clamping.

A periphery of the operation portion 132 defines a circumferential engagement groove 136. The second end of the lever 180 has a second connection portion 182 at least partially surrounding the operation portion 132. The second connection portion 182 has an extension portion at least partially extending into the engagement groove 136, and the extension portion has a size fitting with a size of the engagement groove 136, such that the second connection portion 182 can drive the second instrument assembly 130 to move vertically without affecting the joint rotation of the first instrument assembly 120 and the second instrument assembly 130.

In some embodiments, the second connection portion 182 includes a first arm 183 and a second arm 184 that are spaced from each other and form a forked shape, a claw-like shape, an arched shape, or the like, in order to surround the operation portion 132 between the first arm 183 and the second arm 184. Moreover, the first arm 183 has a first protrusion 185 extending towards the second arm 184, the second arm 184 has a second protrusion 186 extending towards the first arm 183, and each of the first protrusion 185 and the second protrusion 186, as a respective part of the extension portion, extends into the engagement groove 136. In this way, the second instrument assembly 130 can be driven to move vertically stably.

Referring to FIG. 4, for the convenience of determining whether the clamp assembly has been closed, a sensing device 190 may be arranged on the base 110. Accordingly, the lifting assembly 160 is provided with a sensed element 163 arranged on a lateral surface of the lifting assembly 160 and between two legs 171. The sensing device 190 is configured to sense positions of the sensed element 163, and the positions of the sensed element 163 corresponds to states of the jaw 122 and the blade 121.

In some embodiments, the sensing device 190 may be a displacement sensor, and when the sensed element 163 descends to a specific height, it indicates that the clamp assembly has been closed.

In some embodiments, the sensed element 163 is configured as a pressing block, and the sensing device 190 has a pressure switch 191 arranged directly below the pressing block. When the pressing block presses on the pressure switch 191, it indicates that the jaw 122 and the blade 121 have been closed.

The control mechanism 100 of the surgical instrument according to the present disclosure uses a lever structure for transmission between rotation movement and vertical movement. This lever structure is relatively simple, and has low failure rate and low cost. This lever structure results in less redundancy in transmission, which is conducive to precise control of clamping of the ultrasound tool. Moreover, the first transmission disc 141 is arranged to be far away from the ultrasound tool, which is conducive to manual operation of the first transmission disc 141, thereby facilitating the installation and cleaning of the ultrasound tool.

A second embodiment of the present disclosure is a variation of the first embodiment. In the second embodiment, a control mechanism 200 of the surgical instrument has a structure and/or construction similar to that of the control mechanism 100 of the surgical instrument in the first embodiment, except the first connection portion 281 and the cooperation part 261. Therefore, elements with functions that are essentially the same as those in the first embodiment will have the same reference numerals herein, and for the sake of simplicity, they will not be illustrated and/or shown in detail.

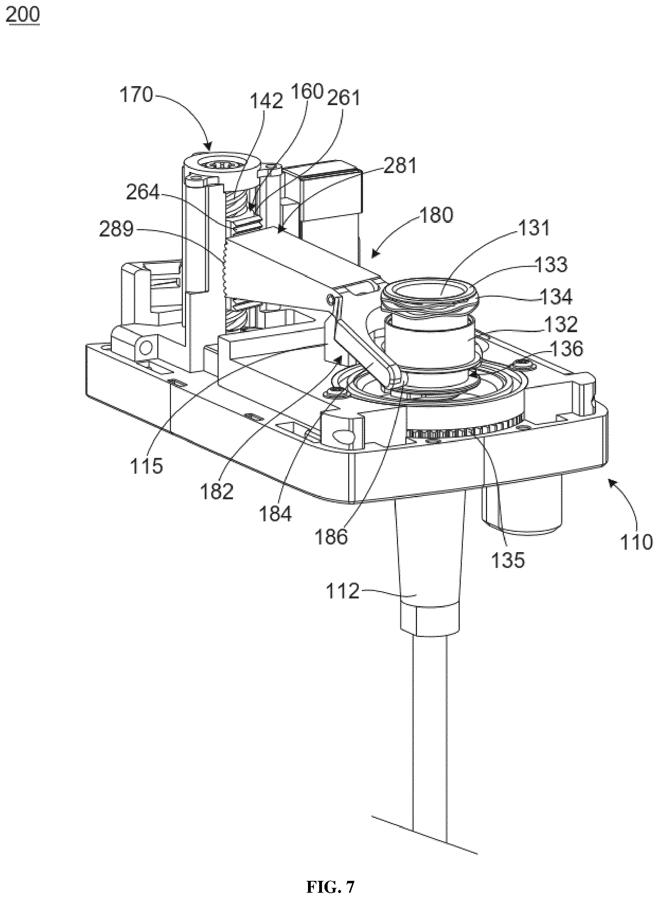

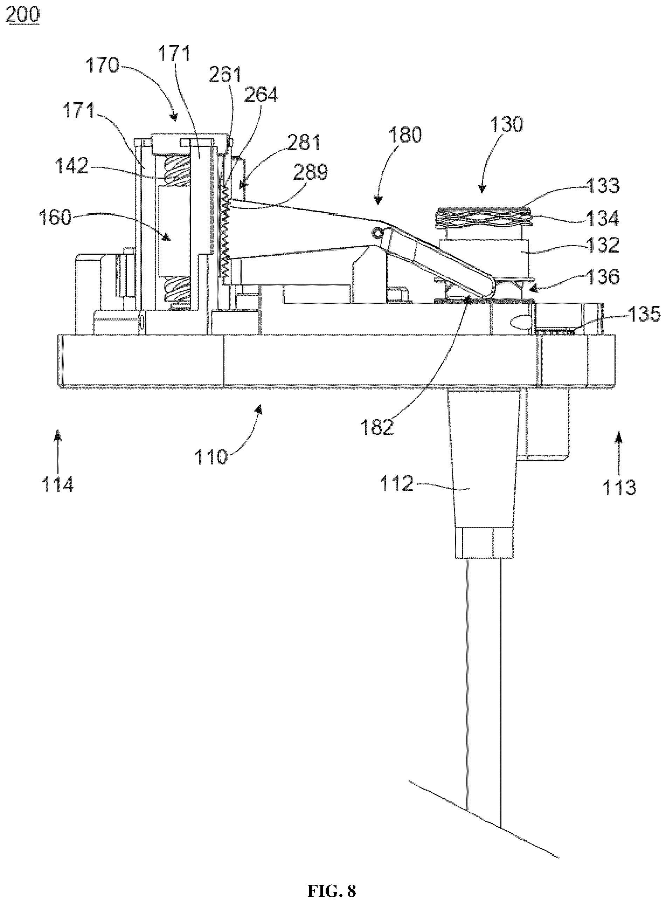

Referring to FIGS. 7 and 8, the cooperation part 261 has first teeth 264 arranged vertically on a side of the cooperation part facing towards the lever 180, in order to form a rack-like structure. The first connection portion 281 of the lever 180 has second teeth 289 arranged on a side of the first connection portion facing towards the lifting assembly 160, the second teeth 289 are arranged on a surface having a circular arc shape centered at the fulcrum of the lever 180. Thus, the second teeth 289 can be regarded as a part of a gear with the fulcrum as a center of circle. Moreover, the first teeth 264 mesh with the second teeth 289 to enable the vertical movement of the first connection portion 281 of the lever 180 with the vertical movement of the lifting assembly 160.

The control mechanism of the surgical instrument according to the present disclosure uses a lever structure for transmission between rotation movement and vertical movement. This lever structure is relatively simple, and has low failure rate and low cost. Moreover, the first transmission disc is arranged to be far away from the ultrasound tool, which is conducive to manual operation of the first transmission disc, thereby facilitating the installation and cleaning of the ultrasound tool.

A third embodiment of the present disclosure is a variation of the first embodiment. In the third embodiment, a control mechanism 300 of the surgical instrument has a structure and/or construction similar to that of the control mechanism 100 of the surgical instrument in the first embodiment, except the rotating rod 342, the cooperation part 361, the first connection portion 381 and the connection parts 388. Therefore, elements with functions that are essentially the same as those in the first embodiment will have the same reference numerals herein, and for the sake of simplicity, they will not be illustrated and/or shown in detail.

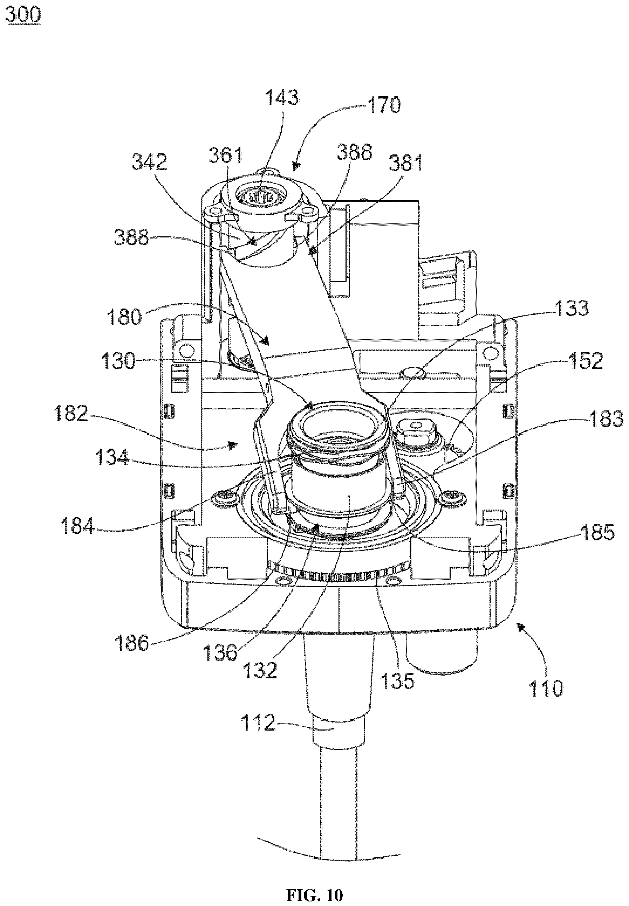

Referring to FIG. 10, in the third embodiment, the lifting assembly 160 is removed, and the rotating rod 342 is configured to have the cooperation part 361 extending circumferentially and spirally. The first end of the lever 180 has the first connection portion 381 configured as a recess for receiving the rotating rod 342. Moreover, the first connection portion 381 are provided with connection parts 388 engaged with the cooperation part 361, and the rotation of the rotating rod 342 renders an upward movement or a downward movement of the first connection portion 381, along the spiral cooperation part 361.

The cooperation part 361 may be configured as a spiral groove, the connection parts 388 may be configured as cylinders extending into the spiral groove, and each of the two branches of the first connection portion 381 is provided with a connection part 388, respectively. In this way, the first connection portion 381 can move vertically stably. By designing tolerances for the cooperation part 361, namely the spiral groove or threads, the horizontal displacement of the first connection portion 381 can be offset.

The control mechanism of the surgical instrument according to the present disclosure uses a lever structure for transmission between rotation movement and vertical movement. This lever structure is relatively simple, and has low failure rate and low cost. Moreover, the first transmission disc is arranged to be far away from the ultrasound tool, which is conducive to manual operation of the first transmission disc, thereby facilitating the installation and cleaning of the ultrasound tool.

A fourth embodiment of the present disclosure is a variation of the first embodiment. The fourth embodiment is different from the first embodiment by: a connection part 188 is arranged on a side of the cooperation part 161, and a kidney-shaped slot 162 is defined on a side of the first connection portion 181 opposite to the connection part 188.

A fifth embodiment of the present disclosure is a variation of the first embodiment. The fifth embodiment is different from the first embodiment by: the control mechanism of the surgical instrument further includes a link arranged between the cooperation part 161 and the first connection portion 181, and each of the cooperation part and the first connection portion is pivotally connected with the link, such that the cooperation part 161 is pivotally connected to the first connection portion 181 via the link, thereby providing the lever 180 with a horizontal movement margin.

A sixth embodiment of the present disclosure is a variation of the first embodiment. The sixth embodiment is different from the first embodiment by: the second connection portion 182 is configured as an annular portion surrounding the operation portion 132. In other words, the annular portion is arranged around the operation portion 132. The annular portion has the extension portion arranged circumferentially, and the extension portion extends into the engagement groove 136. In this way, the transmission of the lever 180 to the second instrument assembly 130 can be more stable.

Another aspect of the present disclosure provides a surgical robot (not shown), the surgical robot includes at least part of the features of the control mechanism 100 of the surgical instrument according to any one of the embodiments as illustrated above, and can achieve similar technical effects.

Unless otherwise defined, the technical and scientific terms used in the present disclosure have the same meanings as those commonly understood by those skilled in the art. The terms used in the present disclosure are only for describing specific implementation purposes and are not intended to limit the present disclosure. The features described in one embodiment in the present disclosure may be applied separately or in combination with other features to another embodiment, unless the features are not applicable in that another embodiment or otherwise specified.

The present disclosure has been illustrated through the above embodiments, but it should be understood that the above embodiments are only exemplary and for illustration, and the present disclosure is not limited to the above embodiments. According to the teachings of the present disclosure, a plurality of variations and modifications can be made, all of which fall within the claimed scope of protection of the present disclosure.

## Claims

1. A control mechanism for a surgical instrument, configured to be connected to an effector of the surgical instrument and configured to control a clamp assembly of the effector to open or close, wherein the control mechanism includes:
a base;
an operation assembly configured to be connected to the effector;
a first transmission assembly arranged on the base and configured to engage with a drive mechanism, wherein the first transmission assembly is rotatable under control of the drive mechanism; and
a lever having a first end, a second end opposite to the first end, and a lever body, wherein the first end of the lever is connected to the first transmission assembly and is movable vertically with rotation of the first transmission assembly, the second end of the lever is connected to the operation assembly, and the lever body of the lever is pivotably connected to the base to form a fulcrum; and
wherein an upward movement of the first end of the lever causes the clamp assembly to open; and
a downward movement of the first end of the lever causes the clamp assembly to close.

2. The control mechanism according to claim 1, wherein the first transmission assembly includes a first transmission disc and a rotating rod having threads, the first transmission disc is rotatably connected to a bottom of the base, the rotating rod is fixed and connected on the first transmission disc, and the first end of the lever is movable with a rotation of the rotating rod; and
wherein the base has a front end and a rear end opposite to each other, a second instrument assembly is arranged at the front end, the first transmission disc is arranged at the rear end, and a notch is defined on the rear end of the base to expose the first transmission disc.

3. The control mechanism according to claim 2, further including a support seat arranged on the base, wherein the support seat is pivotably connected to an upper portion of the rotating rod.

4. The control mechanism according to claim 3, further including a manual control part arranged at a top end of the rotating rod, and the support seat defines an opening corresponding to the manual control part; and
wherein the control mechanism of the surgical instrument further includes a housing covering on the base, and the housing defines a through hole corresponding to the manual control part and the opening.

5. The control mechanism according to claim 2, wherein the first end of the lever has a first connection portion configured as a recess for receiving the rotating rod, the threads of the rotating rod are configured as a cooperation part engaged with a connection part arranged on the first connection portion, and rotation of the rotating rod renders an upward movement or a downward movement of the first connection portion, along spiral cooperation part.

6. The control mechanism according to claim 3, wherein the rotating rod is configured as a threaded rod;
wherein the control mechanism of the surgical instrument further includes a lifting assembly threadedly engaged with the threaded rod, rotation of the threaded rod renders an upward movement or a downward movement of the lifting assembly; and
wherein the first end of the lever is connected to the threaded rod via the lifting assembly.

7. The control mechanism according to claim 6, wherein the first end of the lever has a first connection portion, the lifting assembly includes a cooperation part corresponding to the first connection portion, the cooperation part is movably connected to the first connection portion to provide the lever with a horizontal movement margin.

8. The control mechanism according to claim 7, wherein one of the cooperation part and the first connection portion is configured as a protrusion, and an other of the cooperation part and the first connection portion is configured as a recess fitted with the protrusion;
wherein at least one kidney-shaped slot is defined on a side of one of the cooperation part and the first connection portion, a connection part is arranged on a side of an other of the cooperation part and the first connection portion, and the connection part extends into the at least one kidney-shaped slot to form a hinge connection; and
wherein a length direction of the at least one kidney-shaped slot intersects with a vertical direction to form the horizontal movement margin.

9. The control mechanism according to claim 8, wherein the connection part is configured as a cylindrical rod or a bearing slidable or rollable in the at least one kidney-shaped slot.

10. The control mechanism according to claim 7, further including a link arranged between the cooperation part and the first connection portion, wherein each of the cooperation part and the first connection portion is pivotally connected with the link.

11. The control mechanism according to claim 7, wherein the cooperation part has first teeth arranged vertically, the first connection portion has second teeth, the second teeth are arranged on a surface having a circular arc shape centered at the fulcrum of the lever, and the first teeth mesh with the second teeth.

12. The control mechanism according to claim 7, wherein a guide groove is defined on the support seat and extends vertically, and the cooperation part extends out of the support seat via the guide groove.

13. The control mechanism according to any one of claims 1 to 12, further including a second transmission assembly configured to engage with the drive mechanism, wherein the second transmission assembly is rotatable under control of the drive mechanism; and
wherein the second transmission assembly is configured to be connected to the effector to drive the effector to rotate with the second transmission assembly.

14. The control mechanism according to claim 13, wherein the second transmission assembly includes a second transmission disc and a first gear, the second transmission disc is arranged on a bottom of the base, and the first gear is fixed and connected on the second transmission disc; and
wherein the first gear is configured to mesh with a second gear connected to the effector.

15. The control mechanism according to claim 13, wherein the operation assembly includes an operation portion, and a circumferential engagement groove is defined on a periphery of the operation portion; and
wherein the second end of the lever has a second connection portion at least partially surrounding the operation portion, the second connection portion has an extension portion at least partially extending into the engagement groove, the extension portion has a size fitting with a size of the engagement groove to enable a vertical movement of the operation portion with pivoting of the second connection portion and enable rotation of the operation portion.

16. The control mechanism according to claim 15, wherein the second connection portion includes a first arm and a second arm spaced from each other, the operation portion is arranged between the first arm and the second arm, the first arm has a first protrusion, the second arm has a second protrusion, and each of the first protrusion and the second protrusion at least partially extends into the engagement groove; or
wherein the second connection portion is configured as an annular portion surrounding the operation portion, the annular portion has the extension portion arranged circumferentially, and the extension portion extends into the engagement groove.

17. The control mechanism according to claim 15, wherein the operation assembly further includes:
a traction portion configured to be connected to the effector, wherein the operation portion is sleeved on the traction portion, the operation portion is vertically movable relative to the traction portion;
a limiting portion arranged on a top of the traction portion and on an upper side of the operation portion; and
an elastic part sleeved on the traction portion and arranged between the limiting portion and the operation portion.

18. The control mechanism according to any one of claims 6 to 12, wherein the lifting assembly is provided with a sensed element, the base is provided with a sensing device configured to sense positions of the sensed element, and the positions of the sensed element corresponds to states of the clamp assembly.

19. The control mechanism according to claim 18, wherein the sensed element is configured as a pressing block, the sensing device has a pressure switch, and the pressing block is configured to press on the pressure switch when the clamp assembly is closed.

20. The control mechanism according to any one of claims 1 to 12, wherein the surgical instrument is configured as an ultrasound tool, and a blade and a jaw of the ultrasound tool form the clamp assembly.

21. A surgical robot, comprising the control mechanism according to any one of claims 1 to 20.
